# EUROPÄISCHE PATENTSCHRIFT

(11) **EP 0 826 347 B1**
(45) Veröffentlichungstag und Bekanntmachung des Hinweises auf die Patenterteilung: **11.12.2002**
(21) Anmeldenummer: 97113638.7
(22) Anmeldetag: 07.08.1997
(51) Int. Cl.: A61F 2/34, A61F 2/46

(54) **Gelenkprothese**
Joint prosthesis
Prothèse d'articulation

(30) Priorität: 24.08.1996 DE 19634274; 17.01.1997 DE 19701536
(43) Veröffentlichungstag der Anmeldung: 04.03.1998
(73) Patentinhaber: CERASIV GmbH INNOVATIVES KERAMIK-ENGINEERING, D-73207 Plochingen (DE)
(72) Erfinder: Pfaff, Hans-Georg, 73760 Ostfildern (DE); Kälberer, Hartmut, 73779 Deizisau (DE)
(74) Vertreter: Scherzberg, Andreas, Dr.

(56) Entgegenhaltungen:
- EP-A- 0 237 751
- EP-A- 0 444 381
- EP-A- 0 445 068
- EP-A- 0 586 335
- EP-A- 0 640 324
- EP-A- 0 649 641
- EP-A- 0 655 230
- EP-A- 0 694 294
- WO-A-94/23670
- DE-A- 4 337 936
- FR-A- 2 329 249
- US-A- 5 413 610

## Beschreibung

Die vorliegende Erfindung betrifft eine Gelenkprothese mit einem pfannenförmigen Trägerteil, in dem ein keramischer Einsatz mittels konischer Klemmung festsitzend angeordnet ist.

Bekannt sind Gelenkprothesen, die ein metallisches Trägerteil und einen darin enthaltenen Einsatz aufweisen. Das metallische Trägerteil wird im Knochen des Patienten verankert. Der Einsatz bildet eine verschleißfeste Gleitfläche, gegen die die Gelenkkugel des Knochens oder einer anderen Gelenkprothese drückt. Bei den sogenannten harten Gleitpaarungen besteht zumindestens ein Teil der Gelenkverbindung, zum Beispiel der Einsatz aus Metall. Die Befestigung des metallischen Einsatzes in dem ebenfalls aus Metall bestehenden Trägerteil erfolgt mit Hilfe einer Polyethylen-Kupplung. Die Polyethylen-Kupplung ist durch Mikrorelativbewegungen zum Einsatz hin Verschleiß unterworfen. Dieser Verschleiß ist zwar nicht so gravierend wie Reibungsverschleiß, dennoch wird der Sitz des Einsatzes gelockert und der Abrieb gelangt in das Gewebe und die Gelenkschale.

In US 5 282 864 ist eine Gelenkprothese beschrieben, bei der ein metallischer Einsatz mit Hilfe von Schrauben in einem metallischen Trägerteil verankert ist. Der Einsatz besteht aus einer Kobalt-Chrom-Legierung, während das Trägerteil aus einer Titan-Legierung besteht. Bedingt durch diesen Aufbau der Gelenkprothese besteht die Gefahr von Reibkorrosion und galvanischer Korrosion. Bei Korrosion geben die Metalle toxische Ionen ab, die den Patienten schädigen können.

Um diese Nachteile zu vermeiden, werden keramische Einsätze in die metallischen Trägerteile eingesetzt. Da keramische Werkstoffe nur schwer zu verarbeiten sind, werden sie meist mittels konischer Klemmung in dem Trägerteil befestigt. Ein Beispiel hierfür ist in EP 0 649 641 A2 beschrieben.

Konische Klemmungen für Gelenkprothesen, wobei der Winkel der klemmfläche des Einsatzes verschieden ist von dem Winkel der Klemmfläche des Trägerteiles sind aus den Dokumenten US 5 413 610 und FR 2 329 249 bekannt.

Bei der konischen Klemmung treten in bestimmten Zonen des Einsatzes erhebliche Zugspannungen auf, da die Krafteinleitung nicht kontrollierbar ist. Keramische Werkstoffe sind zwar sehr hart und druckfest, können aber nur begrenzt Zugkräfte aufnehmen. Dies führt dazu, daß insbesondere dünnwandige Keramikeinsätze technisch nicht realisiert werden können.

Der. Erfindung liegt die Aufgabe zugrunde, die Verbindung zwischen dem Trägerteil und dem Einsatz so zu gestalten, daß eine sichere reproduzierbare Verbindung erreicht wird und gleichzeitig das Herauslösen des Einsatzes möglich ist.

Diese Aufgabe wird erfindungsgemäß durch die Merkmale des Patentanspruchs 1 gelöst.

Bei der erfindungsgemäßen Gelenkprothese wird der keramische Einsatz mittels konischer Klemmung in dem pfannenförmigen Trägerteil befestigt. Der Winkel der Klemmfläche des Einsatzes hat einen anderen Wert als der Winkel der Klemmfläche des Trägerteils, so daß der Einsatz nur über einen relativ kleinen Bereich mit dem Trägerteil verbunden ist. Dies ermöglicht eine kontrollierte Kraftübertragung von dem Einsatz auf das Trägerteil. Die axial wirkende Gelenkkraft wird in hohem Maß in umfänglich wirkende Druckspannungen umgesetzt, wobei das Entstehen von Zugspannungen im Einsatz minimiert wird. Die Verbindung zwischen Trägerteil und Einsatz wird im Bereich der Öffnungsseite der Gelenkprothese realisiert. An dem der Öffnungsseite abgewandten Ende berühren sich die Klemmflächen nicht. Dies hat den Vorteil, daß die von der Gelenkprothese aufzunehmende Kraft gleichmäßig über den Umfang verteilt in einem bestimmten Bereich eingeleitet wird.

Die lasttragende Oberfläche des Trägerteils kann aufgerauht werden, so daß zum Beispiel eine Rauhigkeit von 20 µm entsteht. So können Unregelmässigkeiten in der Kraftübertragung, die zum Beispiel durch Formabweichungen im Mikrobereich entstehen, vermieden werden.

Zur Verbesserung der Reibungseigenschaften kann die Klemmfläche des Trägerteils feinstgedreht oder geschliffen sein, so daß die Oberfläche eine Rauhigkeit von 0 bis 4 µm aufweist.

Bevorzugterweise weist der Einsatz an dem der Öffnungsseite abgewandten Ende der Klemmfläche einen Radius oder eine leichte zusätzliche Schräge auf. Dadurch wird ein Verkanten des Einsatzes beim Einführen oder beim Setzen unter Last verhindert werden. Dies kann auch dadurch erreicht werden, daß sich an dem der Öffnungsseite abgewandten Ende der Klemmfläche des Trägerteils ein Freistich anschließt.

Bei Verschleiß des Einsatzes muß dieser ausgewechselt werden. Um die Belastung für den Patienten zu minimieren, wird der Einsatz in situ aus dem Trägerteil entfernt, während dieses am Knochen verbleibt. Um den Einsatz leicht entfernen zu können, ist in einer besonderen Ausgestaltung der Erfindung mindestens eine Ausnehmung vorgesehen, die von der Öffnungsseite an der Grenzfläche zwischen Einsatz und Trägerteil entlang bis zu dem der Öffnungsseite abgewandten Ende der beiden Klemmflächen verläuft. An dem inneren, der Öffnungsseite abgewandten Ende der Ausnehmung befindet sich ein Kipphebel, der die Klemmfläche des Einsatzes hintergreift. Mit einem Werkzeug, wie zum Beispiel einem Keil, kann das in der Ausnehmung befindliche Ende des Kipphebels gegen den Einsatz gedrückt werden, wodurch dieser aus dem Trägerteil entfernt wird.

Im folgenden werden Ausführungsbeispiele der Erfindung unter Bezugnahme auf die Zeichnungen näher erläutert.

Es zeigen:
- Fig. 1: einen Schnitt durch eine Gelenkprothese mit Trägerteil und einem darin festsitzenden Einsatz,
- Fig. 2: eine Detaildarstellung der konischen Klemmung,
- Fig. 3: eine Detaildarstellung des innengelegenen Endes der Klemmfläche des Einsatzes,
- Fig. 4: eine Detaildarstellung des innengelegenen Endes der Klemmfläche des Trägerteils,
- Fig. 5: eine Detaildarstellung der Gelenkprothese mit einer Ausnehmung und einem darin befindlichen Kipphebel zum Herauslösen des Einsatzes, und
- Fig. 6: eine Detaildarstellung der Gelenkprothese mit einem den Kipphebel aktivierenden Werkzeug.

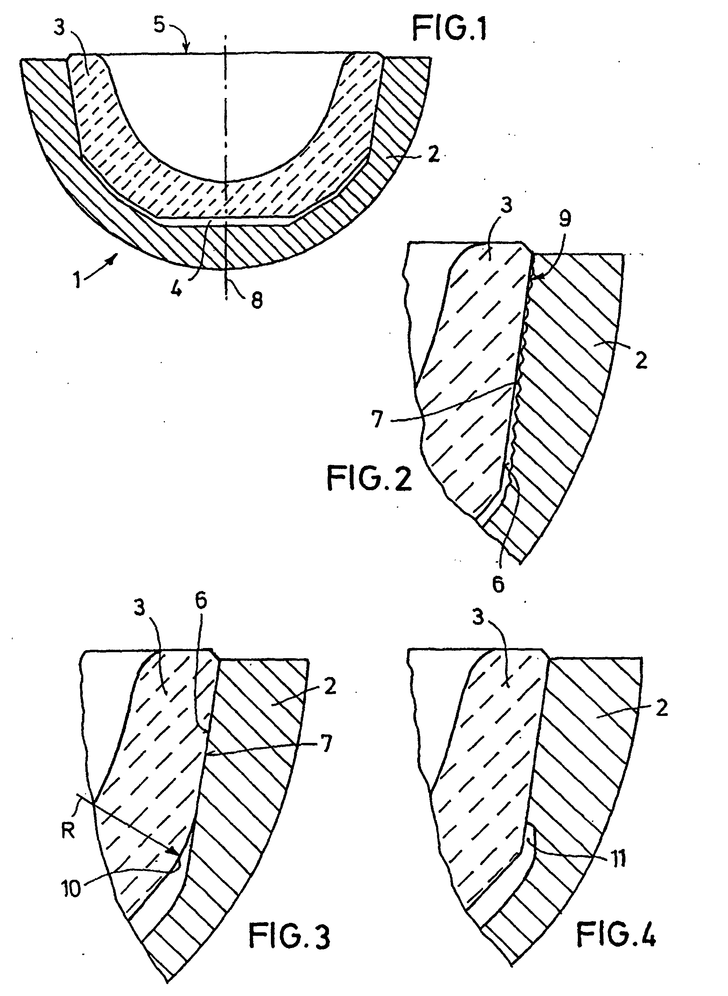

Figur 1 zeigt eine Gelenkprothese 1 bestehend aus einem pfannenförmigen metallischen Trägerteil 2 und einem die künstliche Gelenkschale bildenden keramischen Einsatz 3. Der Einsatz 3 ist in einem Innenraum 4 des Trägerteils 2 angeordnet. Die Innenfläche des Einsatzes 3 ist als Kugelpfanne ausgebildet, die an der Öffnungsseite 5 das Gegenstück des Gelenkes aufnimmt. Der Einsatz 3 wird mittels konischer Klemmung in dem Trägerteil 2 festgehalten.

Die Klemmfläche 6 des Einsatzes 3 verläuft unter einem Winkel α zu der Klemmfläche 7 des Trägerteiles 2 (Figur 2). Der Winkel, unter dem die Einsatz-Klemmfläche 6 zu der Gelenkachse 8 der Gelenkprothese verläuft, ist größer als der Winkel der Trägerteil-Klemmfläche 7.

Dies bewirkt, daß der Einsatz 3 nur in einem relativ kleinen Kontaktbereich 9, der sich an der Öffnungsseite 5 befindet, an das Trägerteil 2 gepreßt wird. Die Größe des Kontaktbereiches 9 kann durch Änderung des Winkels α verändert werden. Je kleiner der Winkel α ist, desto größer wird der Kontaktbereich 9.

Eine besondere Ausführungsart der Erfindung ist in dem abhängigen Patentanspruch 2 angegeben.

Die Klemmfläche 7 des Trägerteiles 2 weist eine Rauhigkeit von etwa 20 µm auf. Dadurch werden Formabweichungen des Einsatzes 3 ausgeglichen.

In den Figuren 3 und 4 sind die Klemmflächen 6 und 7 parallel zueinander und nicht aufgerauht dargestellt, um die Übersichtlichkeit zu verbessern. Tatsächlich erfolgt die Verbindung zwischen dem Einsatz 3 und dem Trägerteil 2 jedoch wie in Fig. 2 dargestellt. In Figur 3 schließt sich an den unteren Endbereich der Klemmfläche 6 des Einsatzes 3 eine gekrümmte Fläche 10 mit dem Radius R an. Bei dem Einführen des Einsatzes 3 in das Trägerteil 2 wird durch diese Abrundung der unteren Kante der Klemmfläche 6 ein Verkanten des Einsatzes 3 in dem Trägerteil 2 vermieden. Dies kann auch dadurch erreicht werden, daß in dem Trägerteil 2 ein Freistich 11 vorgesehen ist, der im Bereich des unteren Endes der Klemmfläche 6 des Einsatzes 3 angeordnet ist (Fig. 4).

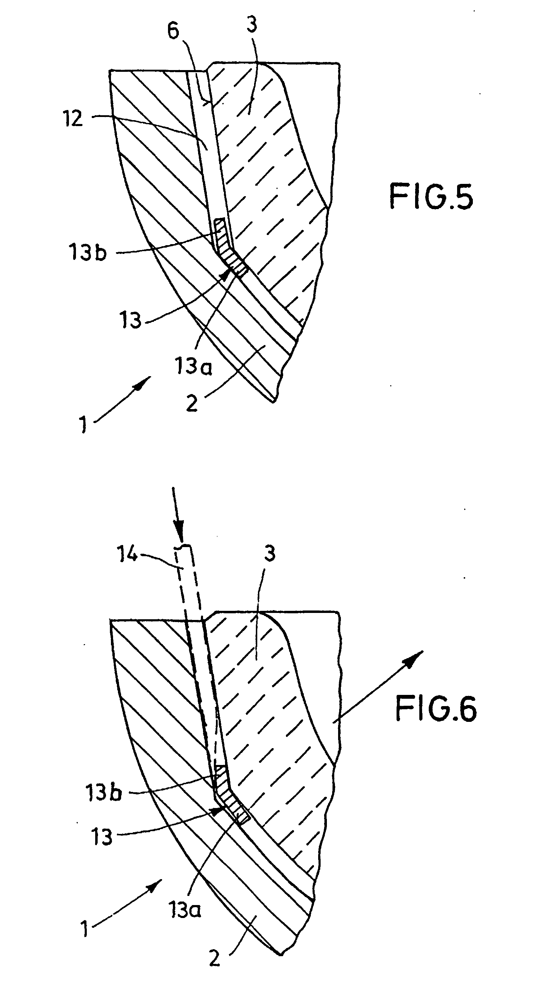

In Figur 5 ist ein weiteres Ausführungsbeispiel der Gelenkprothese 1 dargestellt. Das Trägerteil 2 weist eine längliche Ausnehmung 12 auf, die sich im Grenzbereich zwischen dem Trägerteil 2 und dem Einsatz 3 befindet und parallel zu der Klemmfläche 6 des Einsatzes 3 verläuft. Die Ausnehmung 12 ist zur Öffnungsseite 5 der Gelenkprothese 1 offen. In dem unteren Ende der Ausnehmung befindet sich ein Kipphebel 13, der aus zwei Schenkeln 13a und 13b besteht, die unter einem flachen Winkel zueinander angeordnet sind. Der Schenkel 13a hintergreift die Klemmfläche 6 des Einsatzes 3 und wird zwischen dem Einsatz 3 und dem Trägerteil 2 eingeklemmt. Der zweite Schenkel 13b ragt in die Ausnehmung 12 hinein. Der Winkel zwischen den beiden Schenkeln 13a,13b ist so ausgelegt, daß der zweite Schenkel 13b etwa parallel zu der Ausnehmung 12 verläuft.

In Figur 6 ist gezeigt, wie mit Hilfe eines keilförmigen Werkzeuges 14 der Kipphebel 13 aktiviert wird. Der Keil 14 wird in die Ausnehmung 12 geschoben, bis die Keilspitze sich zwischen dem Trägerteil 2 und dem zweiten Schenkel 13b des Kipphebels 13 befindet. Durch weiteren Druck auf den Keil 14 wird der zweite Schenkel 13b in Richtung des Einsatzes 3 gedrückt. Dies verursacht auch eine Bewegung des ersten Schenkels 13a, der den Einsatz 3 aus dem Trägerteil 2 herausdrückt.

## Patentansprüche

1. Gelenkprothese mit einem pfannenförmigen Trägerteil (2), in dessen Innenraum ein keramischer Einsatz (3) mittels konischer Klemmung festsitzend angeordnet ist, **dadurch gekennzeichnet, dass** der Winkel der Klemmfläche (6) des Einsatzes (3) verschieden ist von dem Winkel der Klemmfläche (7) des Trägerteiles (2), wobei die Klemmfläche (6) des Einsatzes (3) im Bereich der Öffnungsseite (5) der Gelenkprothese an der Klemmfläche (7) des Trägerteiles (2) zur Erzeugung der konischen Klemmung anliegt.

2. Gelenkprothese nach Anspruch 1, **dadurch gekennzeichnet, dass** die Klemmfläche (6) des Einsatzes (3) unter einem Winkel (α) von 1 bis 20 Winkelminuten, bezogen auf die Klemmfläche (7) des Trägerteiles (2), verläuft, vorzugsweise unter einem Winkel (α) von 1 bis 5 Winkelminuten.

3. Gelenkprothese nach Anspruch 1 oder 2, **dadurch gekennzeichnet, dass** die Klemmfläche (7) des Trägerteiles (2) strukturiert ist.

4. Gelenkprothese nach Anspruch 3, **dadurch gekennzeichnet, dass** die Klemmfläche (7) des Trägerteiles (2) eine Rauhigkeit von 20 µm aufweist.

5. Gelenkprothese nach Anspruch 1 oder 2, **dadurch gekennzeichnet, dass** die Klemmfläche (7) des Trägerteiles (2) glatt ist.

6. Gelenkprothese nach Anspruch 5, **dadurch gekennzeichnet, dass** die Klemmfläche (7) des Trägerteiles (2) eine Rauhigkeit von 0 bis 4 µm aufweist.

7. Gelenkprothese nach einem der Ansprüche 1 bis 6, **dadurch gekennzeichnet, dass** der Einsatz (3) an dem Endbereich der konischen Klemmfläche (6) mit dem kleineren Durchmesser einen Radius (R) oder eine leichte zusätzliche Schräge (10) aufweist, die ein Verkanten beim Einsetzen des Einsatzes (3) in das Trägerteil (2) verhindert.

8. Gelenkprothese nach einem der Ansprüche 1 bis 6, **dadurch gekennzeichnet, dass** das Trägerteil (2) an dem dem Innenraum (4) zugewandten Endbereich der Klemmfläche (7) einen Freistich (11) aufweist.

9. Gelenkprothese nach einem der Ansprüche 1 bis 8, **dadurch gekennzeichnet, dass** in dem Trägerteil (2) auf der Klemmfläche (7) zumindestens eine Ausnehmung (12) von der Öffnungsseite (5) zu dem Innenraum (4) verläuft, dass am inneren Enden der Ausnehmung (12) ein die Klemmfläche (6) des Einsatzes (3) hintergreifender Kipphebel (13) angeordnet ist und dass zum Lösen des Einsatzes (3) aus dem Trägerteil (2) der Kipphebel (13) mit einem Werkzeug (14) umlegbar ist.

## Claims

1. Joint prosthesis comprising a cup-shaped carrier part (2), in the interior of which a ceramic insert (3) is disposed in a tight-fitting manner by means of conical clamping, **characterized in that** the angle of the clamping face (6) of the insert (3) differs from the angle of the clamping face (7) of the carrier part (2), with the clamping face (6) of the insert (3) lying in the region of the opening side (5) of the artificial joint against the clamping face (7) of the carrier part (2) so as to produce the conical clamping.

2. Joint prosthesis according to claim 1, **characterized in that** the clamping face (6) of the insert (3) extends, in relation to the clamping face (7) of the carrier part (2), at an angle (α) of 1 to 20 angular minutes, preferably at an angle (α) of 1 to 5 angular minutes.

3. Joint prosthesis according to claim 1 or 2, **characterized in that** the clamping face (7) of the carrier part (2) is structured.

4. Joint prosthesis according to claim 3, **characterized in that** the clamping face (7) of the carrier part (2) has a roughness of 20 µm.

5. Joint prosthesis according to claim 1 or 2, **characterized in that** the clamping face (7) of the carrier part (2) is smooth.

6. Joint prosthesis according to claim 5, **characterized in that** the clamping face (7) of the carrier part (2) has a roughness of 0 to 4 µm.

7. Joint prosthesis according to one of claims 1 to 6, **characterized in that** the insert (3) at the end region of the conical clamping face (6) with a smaller diameter has a radius (R) or a slight additional bevel (10), which prevents tilting during insertion of the insert (3) into the carrier part (2).

8. Joint prosthesis according to one of claims 1 to 6, **characterized in that** the carrier part (2) at the end region of the clamping face (7) directed towards the interior (4) has an undercut (11).

9. Joint prosthesis according to one of claims 1 to 8, **characterized in that**, in the carrier part (2), at least one recess (12) extends from the opening side (5) along the clamping face (7) to the interior (4), that a rocking lever (13), which engages behind the clamping face (6) of the insert (3), is disposed at the inner end of the recess (12) and that, for releasing the insert (3) from the carrier part (2), the rocking lever (13) is relocatable by means of a tool (14).

## Revendications

1. Prothèse d'articulation comportant une partie support (2) en forme de cuvette dans la chambre intérieure de laquelle un insert (3) en céramique est fixé par serrage conique, **caractérisé en ce que** l'angle de la surface de serrage (6) de l'insert (3) est différent de l'angle de la surface de serrage (7) de la partie support (2), 1a surface de serrage (6) de l'insert (3), dans la région du côté ouvert (5) de la prothèse d'articulation, étant en contact avec la surface de serrage (7) de la partie support (2) aux fins de réaliser un serrage cônique.

2. Prothèse d'articulation selon la revendication 1, **caractérisée en ce que** la surface de serrage (6) de l'insert (3) forme un angle (α) compris entre 1 et 20 minutes avec la surface de serrage (7) de la partie support (2), de préférence un angle (α) compris entre 1 et 5 minutes.

3. Prothèse d'articulation selon la revendication 1 ou 2, **caractérisée en ce que** la surface de serrage (7) de la partie support (2) est structurée.

4. Prothèse d'articulation selon la revendication 3, **caractérisée en ce que** la surface de serrage (7) de 1a partie support (2) présente une rugosité de 20 µm.

5. Prothèse d'articulation selon la revendication 1 ou 2, **caractérisée en ce que** la surface de serrage (7) de la partie support (2) est lisse.

6. Prothèse d'articulation selon la revendication 5, **caractérisée en ce que** la surface de serrage (7) de la partie support (2) présente une rugosité allant de 0 à 4 µm.

7. Prothèse d'articulation selon une des revendications 1 à 6, **caractérisée en ce que** l'insert (3), au niveau de la portion d'extrémité de la surface de serrage (6) conique de diamètre plus faible, présente un rayon (R) ou un léger chanfrein additionnel, qui empêche un coincement de l'insert (3) lors de sa mise en place dans la partie support (2).

8. Prothèse d'articulation selon une des revendications 1 à 6, **caractérisée en ce que** la partie support (2), au niveau de la portion d'extrémité de la surface de serrage (7) tournée vers la chambre intérieure (4), présente un dégagement (11).

9. Prothèse d'articulation selon une des revendications 1 à 8, **caractérisée en ce qu'**un évidement (12) qui s'étend du côté ouvert (5) jusqu'à la chambre intérieure (4) est aménagé dans la partie support (2), sur la surface de serrage (7), **en ce qu'**à l'extrémité intérieure de l'évidement (12) est disposé un levier (13) qui s'engage derrière la surface de serrage (6) de l'insert (3) et **en ce que** pour démonter l'insert (3) de la partie support (2) le levier (13) peut être déplacé à l'aide d'un outil (14).
